# EUROPEAN PATENT APPLICATION

(11) **EP 3 156 923 A1**
(43) Date of publication of application: **19.04.2017**
(21) Application number: 15189395.5
(22) Date of filing: 12.10.2015
(51) Int. Cl.: G06F 19/00

(54) **MOLECULAR PROFILE MATCHING OF TUMOURS**

(71) Applicant: OncoDNA SA, 6041 Gosselies (BE)
(72) Inventor: DETIFFE, Jean-Pol, 6500 Beaumont (BE); LAES, Jean-François, 1400 Nivelles (BE); GHITTI, Gregori, 4460 Grâce-Berleur (BE)
(74) Representative: Lesthaeghe, David

(57) **Abstract**

The invention provides for a computer-implemented method designed for matching one or more 'medical twins' to a patient having a disease, more specifically cancer, with a dual purpose of improved disease profiling and an optimization of the treatment of cancer patients provided through healthcare professionals.

## Description

### FIELD OF THE INVENTION

Provided herein is a computer-implemented method for matching one or more medical twins to a patient having a disease.

### BACKGROUND

Cancer, otherwise known as a malignant tumour, is a large disease family with over a 100 different types or variations, sharing characteristics related to abnormal cell growth with the potential to invade or spread to other parts of the body. A difficulty faced by modern health facilities is the great diversity in patient response to cancer treatment methods due to the high variety in their respective molecular pathways. For example, two cancers cells from two patients stemming from a similar organ can show more differences at the molecular level than two cancers cells from two different organs.

At the moment healthcare professionals (such as oncologists) are using several technologies in cancer profiling to better characterize the molecular pathways used by cancer cells to resist the treatments (e.g., fight, change, adapt, move, escape). One of these technologies involves molecular profiling wherein a variety of testing methods is employed to look at each patient's cancer cells and study the genetic characteristics as well as the presence of any unique biomarkers. By learning more about the molecular biology of cancer researchers obtain more and better targets for novel treatments which, along with chemotherapy, drugs or combinations thereof, can facilitate the development of new classes of molecules (e.g., monoclonal antibodies and small signaling pathway inhibitors) to better identify and classify cancer types. Moreover, based on this molecular profiling, healthcare professionals are capable of making better choices to optimize individual patient treatments to combat each cancer profile in a personalized manner to maximize a patient's response to the treatment. However, due to the great diversity in cancer types the vast amount of molecular profiles and treatment methods it can become difficult to properly navigate and customize to each individual patient's needs.

Accordingly, a need arises for other technologies to monitor the total cancer profiles from any biopsies (invasive or not), as well as inform in real time about new discoveries associated with new advances and opportunities in treatment methods for patients. This technology is preferably capable of following these advances, thereby providing healthcare professional with much needed tools to better characterize the specific cancer types of the patients in their care and, in addition, advise them in choosing the best treatment options corresponding to the patients' conditions. Collaboration between health facilities, healthcare professionals, cancer research centers and any other outside facility developing new treatments such as pharmaceuticals companies, would serve to further advance the knowledge of cancer and treatment methods.

The ability of such an intelligent dynamic system to produce satisfactory responses to health care professional's expectations depends on the ability to integrate any medical data, to manage and validate a knowledge database, to present the data and its evolution to the healthcare professionals in a comprehensive way, and finally to build an artificial intelligence implemented by the system capable of sharing a comprehensive knowledge network. A well-designed user interface can improve a user's experience in interacting with the system and promote the user's confidence in the system's services and capabilities. Those technologies are very different and at the moment there exists no system or method to show these results in a very comprehensive way.

### SUMMARY OF THE INVENTION

A computer-implemented method is presented designed for matching one or more 'medical twins' to a patient having a disease, more preferably cancer, with a dual purpose of improved disease profiling and an optimization of the treatment of cancer patients provided through healthcare professionals (such as practitioners, oncologists).

According to a first aspect, the invention comprises a computer-implemented method for matching one or more medical twins to a patient having a disease the method. Preferably, the method comprises the steps of:
- receiving practitioner data on the patient and saving the practitioner data in a practitioner database;
- receiving laboratory data of the patient and saving the laboratory data in a laboratory database;
- receiving monitoring data of the patient and saving the monitoring data in a monitoring database;
- retrieving practitioner data of other patients from the practitioner database;
- retrieving laboratory data of other patients from the laboratory database;
- retrieving monitoring data of other patients from the monitoring database;
- matching the data of the patient to the data of other patients, thereby obtaining one or more medical twins matched to the patient; and
- outputting the one or more medical twins to a user.

In some preferred embodiments, the method comprises the steps of:
- retrieving external data from a knowledge database; and
- matching the data of the patient to the external data.

In some preferred embodiments, the knowledge database comprises external information, for example academic literature, preferably related to the disease.

In some preferred embodiments, the practitioner database comprises information provided by practitioners, for example selected from the group comprising: patient information, patient treatment information, patient disease information, and patient clinical history.

In some preferred embodiments, the laboratory database comprises laboratory results of patients; for example biopsy test results, genetic test results, and PET scans; preferably biopsy test results of the patient.

In some preferred embodiments, the monitoring database comprises blood analysis results, preferably biomarker analysis results.

In some preferred embodiments, the disease is cancer and the practitioner is an oncologist, preferably wherein the user is the practitioner.

In some preferred embodiments, the method comprises the step of:
- providing information related to one or more medical twins from one or more databases to a user; preferably from the knowledge database, practitioner database, laboratory database, and/or monitoring database.

In some preferred embodiments, the outputting step comprises outputting a ranking of at least two medical twins.

In some preferred embodiments, the matching step and outputting steps are updated subject to one or more changes in any one of the databases, preferably in real time.

In some preferred embodiments, the matching step is performed with a clustering algorithm.

In some preferred embodiments, the clustering algorithm comprises an error minimization strategy, preferably a K-means algorithm.

In some preferred embodiments, the method further comprises the step of:
- receiving feedback on the suitableness of the one or more medical twins for the patient from a user.

In some preferred embodiments, the feedback is inputted in a learning algorithm, preferably wherein the learning algorithm is coupled to the parameters of the clustering algorithm.

According to a second aspect, the invention comprises a computer program, or a computer program product directly loadable into the internal memory of a computer, or a computer program product stored on a computer readable medium, or a combination of such computer programs or computer program products, configured for performing the method according to the first aspect of the invention.

### BRIEF DESCRIPTION OF THE DRAWINGS

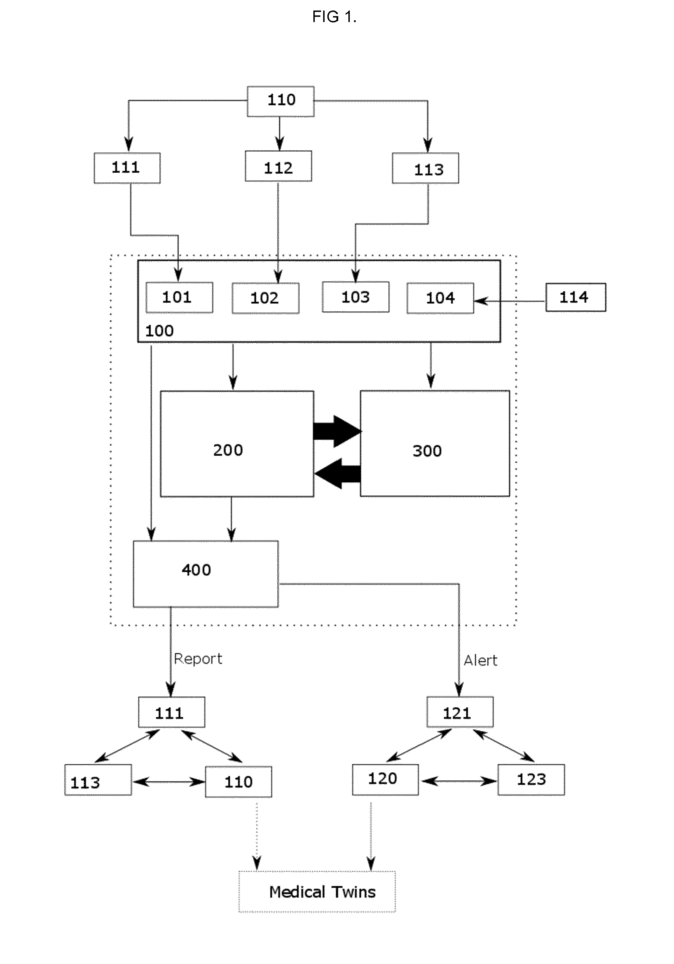

Fig.1: shows a schematic block diagram of the medical twin matching system according to an embodiment of the invention. When patient #1 (110) is diagnosed with the disease his medical data and respective disease profile will be added to the database (100) by the practitioner #1 (111), the laboratory (112), and monitoring devices (113) in charge of said patient's data on, respectively, the practitioner database (101), the laboratory database (102), and the monitoring database (103). Additionally, any external data (114) will be added on the external database (104). Hereafter, a matching engine will run in search of available medical twins stored in the database. As more data is added to the system, the matching engine (200) will become optimized by the learning engine (300). Whenever the matching engine finds medical twins in the system, all practitioners in charge of the medical care of each patient classified as a medical twin will become notified and subsequently presented with the results of said 'successful' matching.

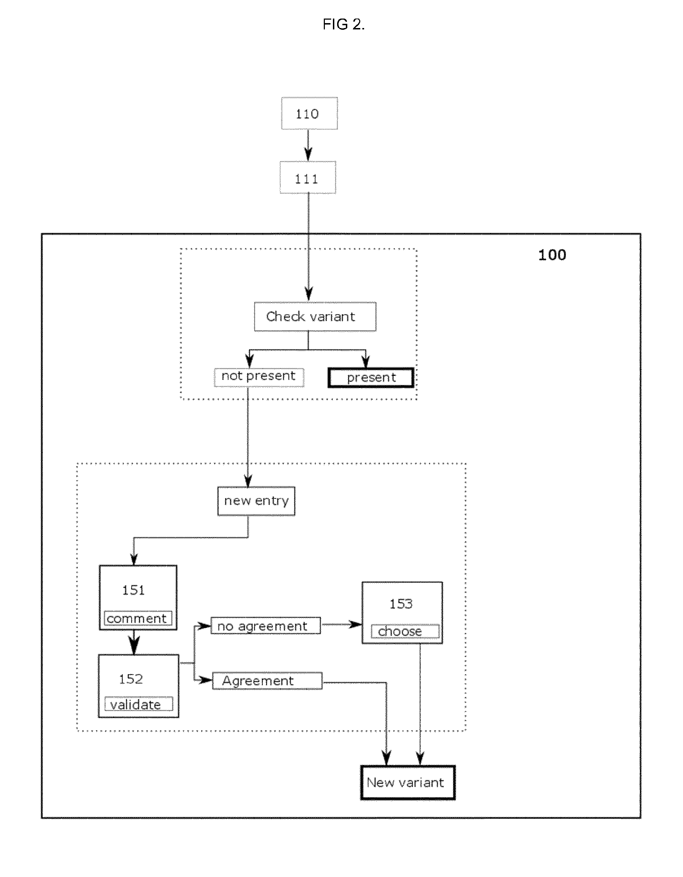

Fig.2: shows a schematic block diagram of a new entry input/check-up system according to an embodiment of the invention. When the patient (110) is diagnosed with the disease, his disease profile will be added to the database (100) by the practitioner (111) charged with his care. The variant of the disease profile will be checked by the system for presence of similar variants: if the disease variant is found to be present the patient entry proceeds to the matching engine (200). However, if the disease variant is not found to be present the disease variant becomes registered as a new entry. The new entry be subjected to a review by a primary person #1 (151) who will comment the new entry, thereafter, both the new entry and the comment will be validated by a second person #2 (152). If an agreement is reached by the first (151) and second person (152) the new entry will proceed to be added as a new disease variant in the system. Should no agreement be reached by the first (151) and second person (152), a third person #3 (153) will be asked to choose the disease variant to be added as a new disease variant into the system.

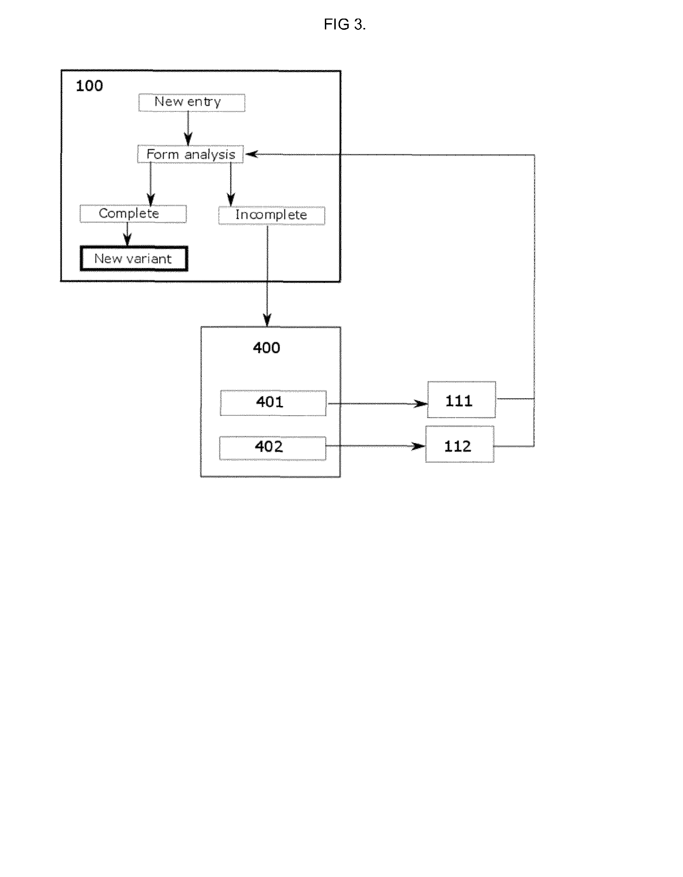

Fig.3: is a schematic block diagram of a new entry verification system according to an embodiment of the invention. When a new entry from an unregistered disease variant is added to the database engine (100) the form will be automatically analyzed by the system for any missing values. If the form is found to be complete the new entry is registered as a new disease variant by the database. However, if the form is found to be incomplete the new entry is forwarded to delivery engine which sends a request to the practitioner (111) using a practitioner interface (401) and/or the laboratory (112) using a laboratory interface (401) in charge of said patient asking to complete the enclosed form regarding the new disease variant. After completion the form is submitted and the process is repeated until the form is found to be completed.

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se*, by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

A computer-implemented method is presented designed for matching one or more 'medical twins' to a patient having a disease, more preferably cancer, with a dual purpose of improved disease profiling and an optimization of the treatment of cancer patients provided through healthcare professionals (such as practitioners, oncologists).

To achieve this purpose, first a database may be set up to contain all the patients' medical data linked to their respective cancer profiles from across several professional healthcare establishments (such as hospitals, labs), preferably entered by the practitioner, laboratory and monitoring devices in charge of said patients' data and healthcare. Thereafter, a series of algorithms starts subdividing the patients into groups based on their medical and disease profiles, thereby enabling a matchmaking search of similar patient types across said healthcare establishments. Patients showing a high level of matching by this system are classified as 'medical twins'.

The primary objective of this matching service will be to provide the practitioners in charge of each respective medical twin with a structured yet user-friendly display of selective features and, if available, an analysis of the cancer profile. This display might be presented with a plurality of information, such as an overview of treatments performed on patients showing similar cancer profiles and the outcomes of the treatments followed. This report would grant practitioners a comprehensive tool for more objective focused disease profiling and optimal treatment selection.

An initial report can be presented whenever a new patient is added to the system consisting of currently available information on its cancer profile. In addition, if in a later stadium in time a different newly added patient is found to match the cancer profile of the initial patient, the system will can alert the practitioner of the prior patient of any new matches and/or about new discoveries associated with novel opportunities in treatment methods as they are entered into the system.

A secondary objective of this system will be the optimization of the grouping algorithm to achieve improved matching results and possible treatment suggestions as more data becomes available. The aim is to feed novel data (for example, from monitoring devices, treatment outcomes/feedback, or better profiling tools,) into a learning engine capable of performing adjustments to the parameters used by the grouping algorithm associated with the ranking of the information, essentially allowing the engine to become a self-learning system.

According to a first aspect, the invention comprises a computer-implemented method for matching one or more medical twins to a patient having a disease the method. Preferably, the method comprises the steps of:
- receiving practitioner data on the patient and saving the practitioner data in a practitioner database;
- receiving laboratory data of the patient and saving the laboratory data in a laboratory database;
- receiving monitoring data of the patient and saving the monitoring data in a monitoring database;
- retrieving practitioner data of other patients from the practitioner database;
- retrieving laboratory data of other patients from the laboratory database;
- retrieving monitoring data of other patients from the monitoring database;
- matching the data of the patient to the data of other patients, thereby obtaining one or more medical twins matched to the patient; and
- outputting the one or more medical twins to a user.

Systems and methods for matching patient profiles, cancer profiles, and cancer treatments profiles are described herein.

As used herein the term "disease" refers to any disease type involving abnormal cell growth with the potential to invade or spread to other parts of the body as is defined by healthcare professionals. These comprise the terms cancer, malignant tumors, and malignant neoplasm.

As used herein the term "patient" refers to any person undergoing medical care or treatments related to issues resulting from the disease, or having done so in the past regardless of the outcome.

As used herein the term "practitioner" refers to any healthcare professional performing any medical care or any treatment on patients suffering from issues related to the disease, or having performed these tasks in the past. These comprise but are not limited to oncologists, primary caretakers, nursing staff, medical staff and health scientists.

As used herein the term "laboratory" refers to any institute or facility performing any scientific measurements related to the disease profiling and research, or having performed these tasks in the past. These comprise but are not limited to pharmaceutical and biotechnology companies, academic and private research centers, and universities.

As used herein the term "medical twin" refers to any matching obtained to any degree as determined by the system or methods presented herein.

As used herein the term "data" refers to any set of values of qualitative or quantitative variables that can be measured, collected, analyzed and reported.

As used herein the term "database" refers to any system capable of storing and organizing a collection of the data.

In some preferred embodiments, the method comprises the steps of:
- retrieving external data from a knowledge database; and
- matching the data of the patient to the external data.

In some preferred embodiments, the knowledge database comprises external information, for example academic literature, preferably related to the disease.

In some preferred embodiments, the practitioner database comprises information provided by practitioners, for example selected from the group comprising: patient information, patient treatment information, patient disease information, and patient clinical history.

In some preferred embodiments, the laboratory database comprises laboratory results of patients; for example biopsy test results, genetic test results, and PET scans; preferably biopsy test results of the patient.

In some preferred embodiments, the monitoring database comprises blood analysis results, preferably biomarker analysis results.

In some embodiments, the monitoring database comprises data obtained from patient monitoring devices. Preferably, the software of patient monitoring devices is accessed.

In some preferred embodiments, the disease is cancer and the practitioner is an oncologist, preferably wherein the user is the practitioner.

In some preferred embodiments, the method comprises the step of:
- providing information related to one or more medical twins from one or more databases to a user; preferably from the knowledge database, practitioner database, laboratory database, and/or monitoring database.

In some preferred embodiments, the outputting step comprises outputting a ranking of at least two medical twins.

The feedback on the matching of the twin may consist in first a percentage of similarity based on a clustering analysis. Then a table comparing every information one by one may be available with the ranking value to provide the practitioner with the possibility to look more into details.

| | | | | |
|---|---|---|---|---|
| Percentage of Similarity | | | 95% | |
| | | | | |

| **Information content** | **ranking** | **Percentage of similarity** | | **Discrepancy with matched patient** |
|---|---|---|---|---|
| Breast cancer ER + / HER2 - | 1 | 100 % | | NA |
| Tamoxifen 4 mg since 6 months | 2 | 100 % | | NA |
| Resistant with ESR1 mutation | 3 | 100 % | | NA |
| ......... | ... | | | |
| p4EPB1 (positive) | 10 | 0% | | Negative |

In some preferred embodiments, the matching step and outputting steps are updated subject to one or more changes in any one of the databases, preferably in real time.

A similar approach that is used in commercial search engines may be used, where relevance of documents to a query changes over time, such as ranking recent documents for a breaking news query. In some embodiments, the user can provide real time feedback, for example by clicking. The information of the feedback as "click information" may be used to determine the pertinence of the information and therefore adjust the rank, preferably after having tested that this new rank will not statistically affect the validated rank information based on the other feedbacks.

In some preferred embodiments, the matching step is performed with a clustering algorithm.

In some preferred embodiments, the clustering algorithm comprises an error minimization strategy, preferably a K-means algorithm.

Preferably, the KNN or k-nearest neighbor's algorithm is used, where new data are classified based on stored, labeled instances. More specifically, the distance between the stored data and the new instance may be calculated by means of some kind of a similarity measure. This similarity to the data that was already in the system can be calculated for any new data point that is input into the system. Then, this similarity value may be used to perform predictive modeling. Predictive modeling is either classification, assigning a label or a class to the new instance, or regression, assigning a value to the new instance. This iterative process may be performed after every supplementary information.

In some preferred embodiments, the method further comprises the step of:
- receiving feedback on the suitableness of the one or more medical twins for the patient from a user.

In some preferred embodiments, the feedback is inputted in a learning algorithm, preferably wherein the learning algorithm is coupled to the parameters of the clustering algorithm.

According to a second aspect, the invention comprises a computer program, or a computer program product directly loadable into the internal memory of a computer, or a computer program product stored on a computer readable medium, or a combination of such computer programs or computer program products, configured for performing the method according to the first aspect of the invention. As used herein, the term "computer system" refers to a computer program, or a computer program product directly loadable into the internal memory of a computer, or a computer program product stored on a computer readable medium, or a combination of such computer programs or computer program products configured for performing the method according to the first aspect of the invention.

In some embodiments, the invention comprises a computer system for matching one or more medical twins to a patient having a disease comprises a database engine (100 - input/entry/storage), a matching engine (200 - KDO - data sorting), a learning (300 - data management) engine, and a delivery (400: retrieving/output) engine.

In some embodiments, the invention comprises a computer system with a database engine (100) for storing and managing a plurality of patient information for matching one or more medical twins comprises a practitioner database (101), a laboratory database (102), a monitoring database (103) and an external database (104).

In some embodiments, the invention comprises the step of submitting a subset of patient information from practitioners and storing it into a practitioner database (101). In such embodiments, the entry can include information to determine the patient's disease profile, for example the symptoms shown by the patient, the patient treatments information from a plurality of traditional and novel treatment options, for example the applied drugs and therapies. In such embodiments, the entry can include other information not related to the cancer profile, but related to healthcare parameters that may have an impact on the proceedings of the matching engine for optimal twin matching and for optimal treatment choices, for example the patient's sex, age, race, and the patient's clinical history. In such embodiments, the entry data can be received from a plurality of sources; these comprise but are not limited to the patients, the practitioners and the laboratories.

In some embodiments, the invention comprises the step of submitting a subset of laboratory information into a laboratory database (102). In such embodiments, the entry can include information to determine the patient's disease profile, for example biomarker results, biopsy test results, genetic test results, sequencing results, and results from patient scans (PET). In such embodiments, the entry data can be received from a plurality of sources; these comprise but are not limited to the laboratory and healthcare professionals and automated entries from said laboratories.

In some embodiments, the invention comprises the step of submitting a subset of monitoring information into a monitoring database (103). In such embodiments, the entry can include information to determine the patient's response to cancer treatments and general patient health status, for example the software applications running on devices used to monitor the patient. In such embodiments, the entry data can be received from a plurality of sources; these comprise but are not limited to the patient, to any (technological) devices connected to the monitoring database, through any software connected to the monitoring database, and any automated entries from any external database.

In some embodiments, the external database (104) refers to any data obtained from outside sources not included in the (101) practitioner database, the (102) laboratory database and the (103) monitoring database, for example, academic literature, the internet ('cloud') and cancer profiling and research related databases (for example genome projects). The external data can be received from automated entries or from human interfaces.

In some embodiments, the matching engine (200) includes any data management action performed on the data stored on the (100) database with the intent of matching one or more medical twins to a patient having cancer. The actions comprise of methods, systems, and algorithms for integrating the external data, integrating the data generated internally, to split the information in sections, to summarize in time frame the relevant information. These actions can be performed by automated data management software run by specifically designed grouping algorithms (Clustering algorithm) or human input performed by reviewers.

In some embodiments the clustering algorithms will group (or cluster) the patients in such a way that the patients in the same group are more similar (in a medical way/disease profile way) to each other than to those in other groups (or clusters).

In a preferred embodiment a k-means algorithm will represent each group with a single mean vector, by partitioning n patients (entries) into k groups (clusters) in which each patients belongs to the cluster with the nearest mean, serving as a prototype of the groups (cluster). In some embodiments, the organization of the patients may be nonhierarchical.

In some embodiments the matching system algorithms and management actions will be adjusted through the outcomes of the follow-up provided by the learning engine (300) and modified rankings of the collected information.

In some embodiments, the learning engine (300) includes any data management action performed on the data stored on the (100) database with the intent of optimizing the matching algorithm run by the matching engine (200). This iterative process will feed the matching engine to improve the ranking of the information by adjusting the parameters for the grouping (clustering) algorithm associated with the ranking of the information.

In some embodiments an error minimization algorithms will search for a clustering structure that minimizes a certain error criterion by measuring the "distance" of each instance (data/group) to its representative value. In some embodiments the learning engine will be combined with additional data entry actions, for example the monitoring of the tumor, to improve the 'efficiency' of the matching engine.

In a preferred embodiment the Sum of Squared Error (SSE) measures the total squared Euclidian distance of instances to their representative values.

In some embodiments, the delivery engine (400) includes any action made to access and deliver the data stored on the practitioner database (101), laboratory database (102) and monitoring database (103) for a plurality of reasons by automated entries, including but not limited to the matching engines, and by human interfaces, including but not limited to healthcare professionals. In an exemplary embodiment these includes requests to complete incomplete (medical) forms to practitioners, the reporting of a medical twin matchings to their respective practitioners (linked to the patient profile) and updates on novel treatment methods.

In preferred embodiments the forms will comprise structured, yet flexible and user-friendly, displays of selective features and/or analysis that allow practitioners more objective focused disease profiling and optimal treatments selections.

## Claims

1. A computer-implemented method for matching one or more medical twins to a patient having a disease the method comprising the steps of:
- receiving practitioner data on the patient and saving the practitioner data in a practitioner database;
- receiving laboratory data of the patient and saving the laboratory data in a laboratory database;
- receiving monitoring data of the patient and saving the monitoring data in a monitoring database;
- retrieving practitioner data of other patients from the practitioner database;
- retrieving laboratory data of other patients from the laboratory database;
- retrieving monitoring data of other patients from the monitoring database;
- matching the data of the patient to the data of other patients, thereby obtaining one or more medical twins matched to the patient; and
- outputting the one or more medical twins to a user.

2. The computer-implemented method according to claim 1, further comprising the steps of:
- retrieving external data from a knowledge database; and
- matching the data of the patient to the external data.

3. The computer-implemented method according to claim 2, wherein the knowledge database comprises external information, for example academic literature, preferably related to the disease.

4. The computer-implemented method according to any one of the preceding claims, wherein the practitioner database comprises information provided by practitioners, for example selected from the group comprising: patient information, patient treatment information, patient disease information, and patient clinical history.

5. The computer-implemented method according to any one of the preceding claims, wherein the laboratory database comprises laboratory results of patients; for example biopsy test results, genetic test results, and PET scans; preferably biopsy test results of the patient.

6. The computer-implemented method according to any one of the preceding claims, wherein the monitoring database comprises blood analysis results, preferably biomarker analysis results.

7. The computer-implemented method according to any one of the preceding claims, wherein the disease is cancer and the practitioner is an oncologist, preferably wherein the user is the practitioner.

8. The computer-implemented method according to any one of the preceding claims, wherein the method comprises the step of:
- providing information related to one or more medical twins from one or more databases to a user; preferably from the knowledge database, practitioner database, laboratory database, and/or monitoring database.

9. The computer-implemented method according to any one of the preceding claims, wherein the outputting step comprises outputting a ranking of at least two medical twins.

10. The computer-implemented method according to any one of the preceding claims, wherein the matching step and outputting steps are updated subject to one or more changes in any one of the databases, preferably in real time.

11. The computer-implemented method according to any one of the preceding claims, wherein the matching step is performed with a clustering algorithm.

12. The computer-implemented method according to claim 11, wherein the clustering algorithm comprises an error minimization strategy, preferably a K-means algorithm.

13. The computer-implemented method according to any one of the preceding claims, further comprising the step of:
- receiving feedback on the suitableness of the one or more medical twins for the patient from a user.

14. The computer-implemented method according to claim 13, wherein the feedback is inputted in a learning algorithm, preferably wherein the learning algorithm is coupled to the parameters of the clustering algorithm.

15. A computer program, or a computer program product directly loadable into the internal memory of a computer, or a computer program product stored on a computer readable medium, or a combination of such computer programs or computer program products, configured for performing the method according to any one of claims 1 to 14.
